# EUROPEAN PATENT SPECIFICATION

(11) **EP 0 930 077 B1**
(45) Date of publication and mention of the grant of the patent: **16.04.2003**
(21) Application number: 98310562.8
(22) Date of filing: 22.12.1998
(51) Int. Cl.: A61K 47/48, A61K 31/366, A61P 33/10

(54) **Cyclodextrin compositions comprising an avermectin or a milbemycin derivative**
Zyklodextrin-Zusammensetzung enthaltend ein Avermectin- oder ein Milbemycin-Derivat
Composition de cyclodextrine contenant un dérivé d'avermectin ou de mibemycin

(30) Priority: 20.01.1998 GB 9801109
(43) Date of publication of application: 21.07.1999
(73) Proprietor: PFIZER INC., New York, N.Y. 10017-5755 (US)
(72) Inventor: Ringshaw, David, Billingshurst, West Sussex RH14 9BL (GB); On, Ninh, Woolwich, London SE18 6AG (GB); Moss, Philip Edward, Sandwich, Kent CT13 9NJ (GB)
(74) Representative: Atkinson, Jonathan David Mark

(56) References cited:
- EP-A- 0 750 907
- THORSTEINN LOFTSSON ET AL: "Pharmaceutical Applications of Cyclodextrins. 1. Drug Solubilization and Stabilization" JOURNAL OF PHARMACEUTICAL SCIENCES, vol. 85, no. 10, 1 October 1996, pages 1017-1025, XP002080430
- THORSTEINN LOFTSSON: "Effect of cyclodextrins on the chemical stability of drugs in aqueous solutions" DRUG STABILITY, vol. 1, no. 1, 1 January 1995, pages 22-33, XP002080429

## Description

The present invention relates to pharmaceutical compositions, in particular to compositions having broad spectrum anthelmintic activity, and their use in human and veterinary medicine.

The use of cyclodextrins as complexing agents for active substances is known and has been previously described in, for example, US-A-4,727,064 to Pitha (amorphous drug/cyclodextrin complexes), EP-A-0 149 197 to Janssen (inclusion complexes of medicaments with a b-cyclodextrin ether or ester) and EP-A-0 392 608 to Procter & Gamble (solid consumer product compositions containing small particle size cyclodextrin complexes).

It has now been found that cyclodextrins are useful for stabilizing certain anthelmintic compounds.

The invention provides a pharmaceutical composition comprising at least one anthelmintically active compound which is an avermectin or milbemycin, in the form of a complex with at least one cyclodextrin. It is believed that the avermectins and milbemycins form inclusion complexes with the cyclodextrins and such inclusion complexes therefore form a particular aspect of the invention.

When used herein the term "pharmaceutical" includes the term "veterinary" and the term "pharmaceutically" includes the term "veterinarily".

Suitable avermectins and milbemycins for use in carrying out the present invention include commercially available compounds such as milbemycin, ivermectin, doramectin, moxidectin, nemadectin, and abamectin. Further suitable compounds are of partial formula (i) 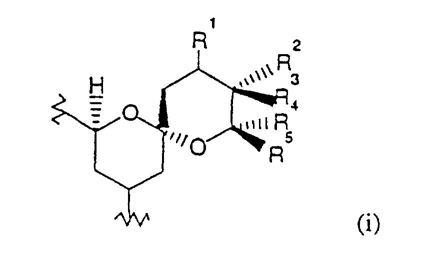 wherein R¹ is an optionally substituted amino or imino group such as optionally O-substituted oxyimino, optionally N-substituted hydrazone or optionally N-substituted semicarbazone, and R² to R⁵ are the same or different and each is hydrogen or an organic radical.

Preferred compounds of formula (i) are compounds of formula (I): 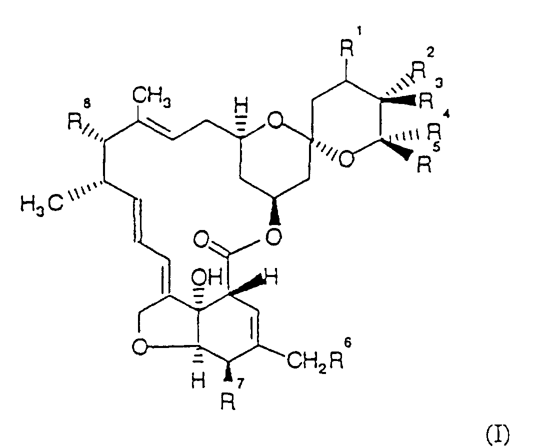 wherein R¹ to R⁵ are as defined above, R⁶ is hydrogen or optionally protected hydroxy; R⁷ is alkoxy, optionally protected hydroxy, oxo or optionally O-substituted oxyimino; and R⁸ is hydrogen, optionally protected hydroxy, or a group 4'-(a-L-oleandrosyl)-a-L-oleandrosyloxy or a-L-oleandrosyloxy wherein the terminal hydroxy group is optionally protected.

Compounds of formula (I), and processes for their preparation, are described in EP-A-0 259 779, EP-A-0 293 549, EP-A-0 307 225, GB-A-2 192 630, EP-A-0 260 536, EP-A-0 260 537, EP-A-0 307 220, and EP-A-0 421 568.

Preferably, the compound of formula (I) is a compound in accordance with EP-A-0 421 568 more especially a compound wherein R¹ is O-substituted oxyimino, R² to R⁴ are hydrogen, R⁵ is an organic radical, R⁶ and R⁸ are hydrogen, and R⁷ is hydroxy.
Suitable avermectins are those of EP-A-0677054, particularly 5-oximino-22,23-dihydro-25-cyclohexylavermectin B1 monosaccharide (Example 5).

Suitable protecting groups for hydroxy include TBDMS (t-butyldimethylsilyl), and acyl (alkanoyloxy). Further suitable protecting groups are described in, for example, "Protective Groups in Organic Synthesis" Theodora W. Greene, Wiley-Interscience 1981 Ch 2, 10-86.

When any of R² to R⁵ is an organic radical it may advantageously be selected from the group consisting of alkyl, alkenyl, alkynyl, aryl, heterocyclyl, mono-, bi- and tri-cycloalkyl, mono-, bi- and tri-cycloalkenyl and aralkyl.

As used herein alkyl includes straight and branched C₁₋₂₀, more especially C₁₋₁₂, particularly C₁₋₆ alkyl, and alkenyl and alkynyl include straight and branched C₂₋₂₀, more especially C₂₋₁₂, particularly C₂₋₆ alkenyl and alkynyl.

When any of R² to R⁵ comprises an alkyl, alkenyl or alkynyl moiety that moiety may optionally be substituted by one or more substituents selected from the group consisting of hydroxy, alkoxy, alkylthio, oxo, halogen, trifluoromethyl, and optionally substituted amino.

When used herein the term 'aryl' includes phenyl and naphthyl optionally substituted with up to five, preferably up to three, groups selected from halogen, C₁₋₆ alkyl, aryl, C₁₋₆ alkoxy, halo substituted (C₁₋₆) alkyl, hydroxy, amino, nitro, carboxy, C₁₋₆ alkoxycarbonyl, C₁₋₆ alkoxycarbonyl-(C₁₋₆)-alkyl, C₁₋₆ alkylcarbonyloxy, or C₁₋₆ alkylcarbonyl groups.

The term 'heterocyclyl' includes saturated, unsaturated and aromatic single or fused rings comprising up to four hetero atoms in the ring selected from oxygen, nitrogen and sulphur and optionally substituted with up to three halogen, C₁₋₆ alkyl, C₁₋₆ alkoxy, halo-(C₁₋₆)-alkyl, hydroxy, amino, carboxy, C₁₋₆ alkoxycarbonyl, C₁₋₆ alkoxycarbonyl(C₁₋₆) alkyl, aryl or oxo groups.

Suitably the heterocyclic ring comprises from 4 to 7 ring atoms, preferably 5 to 6 atoms.

The term 'halogen' refers to fluorine, chlorine, bromine and iodine.

Particularly suitable substituents for an amino or imino group such as an oxime, hydrazone or semicarbazone group include one or more organic radicals as defined hereinabove for R² to R⁵, for example the substituents set out in EP-A-0 288 205, EP-A-0 259 779, EP-A-0 260 537, EP-A-0 260 536, GB-A-2 192 630, EP-A-0 307 225 and EP-A-0 421 568.

Those skilled in the art will appreciate that an N-substituted imino group such as an oxime may exist as either an E or Z isomer, or as a mixture of E and Z isomers, and that an E or Z isomer may be converted to the other isomer or to a mixture of isomers by standard techniques such as acid treatment.

As used herein mono-, bi- and tri-cycloalkyl include C₃₋₂₀, especially C₃₋₁₂, more especially C₄₋₈, groups, and mono-, bi- and tri-cycloalkenyl include C₄₋₂₀, especially C₄₋₁₂, more especially C₅₋₈ groups. When any of R² to R⁵ comprises a mono-, bi- or tri-cycloalkyl or mono-, bi- or tri-cycloalkenyl moiety, that moiety may be substituted as set out above for alkyl, alkenyl, and alkynyl, and/or by one or more substituents selected from the group consisting of methylene and alkyl. Bicyclic and tricyclic groups may be fused or bridged and are preferably attached via a carbon atom which is common to two rings.

Any two of R² to R⁵ may be taken together with the carbon atom(s) to which they are attached to designate a cycloalkyl, cycloalkenyl, aryl or heterocyclyl group which may optionally be substituted as set out above.

Cyclodextrins for use in carrying out the present invention include substituted and unsubstituted cyclodextrins containing from six to twelve glucose units, such as a-, b- and g-cyclodextrins, their derivatives, and mixtures thereof. Derivatives include C₁₋ ₆ alkyl and hydroxy C₁₋₆ alkyl ethers, such as methyl b-cyclodextrin, hydroxyethyl b-cyclodextrin, and hydroxypropyl b-cyclodextrin, as well as polymers and copolymers such as b-cyclodextrin/epichlorohydrin copolymers. Substituted cyclodextrins may exhibit differing degrees of substitution, and may be amorphous or crystalline. Unsubstituted cyclodextrins are usually crystalline.

Cyclodextrins are described in *inter alia* US-A-3,426,011, US-A-3,453,257, US-A-3,459,731, US-A-3,553,191, US-A-3,565,887, US-A-4,535,152, US-A-4,616,008, US-A-4,638,058, US-A-4,746,734, and US-A-4,678,598. Cyclodextrins and mixtures of cyclodextrins are commercially available from *inter alia* Amaizo, Hammond, Indiana, USA; Roquette Corporation, Gurnee, Illinois, USA; Chinoin Pharmaceutical and Chemical Works Ltd., Budapest, Hungary; Aldrich Chemical Company, Milwaukee, Wisconsin, USA; Wacker Chemie, Germany; and Ensuiko Sugar Refining Company, Yokohama, Japan.

In one aspect, the cyclodextrin for use in the present invention is a crystalline cyclodextrin such as b-cyclodextrin or a pure substituted cyclodextrin such as dimethyl b-cyclodextrin, which is commercially available.

The pharmaceutical composition of the invention is of use in the treatment of helminthiasis of the human or non-human animal body, and particularly for treating nematode infestations of domestic and farm animals.

Accordingly, the present invention also provides for the use of an anthelmintically effective amount of a pharmaceutical composition of the invention for the preparation of a medicament for the treatment of helminthiasis, particularly nematode infestations in domestic animals.

Accordingly the present invention also provides a pharmaceutical composition, as hereinbefore defined, (hereinafter called "the composition") for use in the treatment of the human or non-human animal body, especially for treating helminthiasis and particularly for treating nematode infestations of domestic animals, especially dogs and cats. Particular nematode infestations for treatment include trichuris, toxascaris and ancylostoma.

Suitably, the composition may comprise a shaped composition, such as a bolus, tablet or capsule, or can be mixed directly with animal foodstuffs. Optionally the composition contains one or more lubricants, dispersants, binders, fillers, colours, flavours, and the like. Preferably the composition is a tablet which has been rendered more palatable by the inclusion of a food extract such as desiccated liver or fish meal.

The composition may also be added to animal feed. It will be convenient to formulate these animal feed compositions with a multi-dose of the anthelmintic drug so that the animal takes in an appropriate quantity of the composition along with its diet. It will also be convenient to present the composition as a premix for addition to the feed.

The composition may optionally additionally comprise further active ingredients such as further anthelmintic compounds, more especially at least one compound with activity against tapeworm such as *Taenia taeniaeformis* and *Dipylidium caninum.* Such compounds include compounds of formula (II) 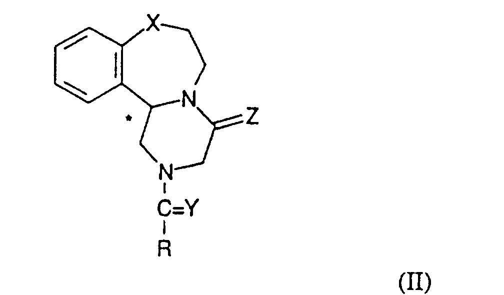 in which R is optionally substituted phenyl; C₃₋₈ cycloalkyl; C₅₋₈ cycloalkenyl; C₁₋₈ alkyl which may be straight or branched; C₂₋₈ alkenyl which may be straight or branched; 5- or 6- membered heterocyclyl; or optionally substituted phenyl C₁₋₄ alkyl, each of Y and Z, which may be the same or different, is oxygen or sulphur; and X is a bond, -CH₂-, or oxygen.

Compounds of formula (II) wherein X is -CH₂- or oxygen, and processes for their production, are described in EP-A-0 134 984 and EP-A-0 187 012. An exemplary such compound is epsiprantel (2-(cyclohexylcarbonyl)-4-oxo-1,2,3,4,6,7,8,12b-octahydropyrazino[2,1-a][2]benzazepine).

Compounds of formula (II) wherein X is a bond, and processes for their production, are described in DE-A-1 795 728, DE-A-24 41 261, DE-A-23 62 539 and by Andrews et al in Medicinal Research Reviews (John Wiley & Sons, Inc.) Vol.3, No. 2, 147-200 (1983). An exemplary such compound is praziquantel (2-cyclohexylcarbonyl[1,2,3,6,7,11b]hexahydro-4H-pyrazino[2,1-a]isoquinolin-4-one).

Compounds of formula (II) have an asymmetric carbon atom marked by an asterisk in formula (II) and may therefore exist in at least two stereoisomeric forms. The present invention encompasses all isomers of the compounds of formula (II) whether pure or admixed with other isomers in any proportion.

When R is optionally substituted phenyl, it may be substituted with one or more moieties selected from halogen, C₁₋₆ alkyl, C₁₋₆ alkoxy, nitro, amino, mono-or-di-C₁₋₆ alkylamino, and hydroxy.

When R is heterocyclyl, it may be a 5 or 6-membered saturated or unsaturated group containing up to three hetero-atoms selected from oxygen, sulphur and nitrogen. It will be appreciated that unsaturated heterocyclyl groups suitably include aromatic heterocyclyl groups.

The term "halogen" refers to fluorine, chlorine, bromine and iodine.

A preferred R group is cyclohexyl.

Compounds of formula (I) have anthelmintic activity especially against tapeworm such as *Taenia toeniaeformis* and *Dipylidium caninum.*

Suitably the composition comprises of sufficient material to provide a dose of from 0.001 to 100 mg of the avermectin or milbemycin compound(s) per kg of animal body weight per dose, more suitably 0.01 to 10mg/kg per dose. Suitably the ratio of avermectin/milbemycin(s) : cyclodextrin(s) is in the range 1 : 1 to 1 : 10 preferably about 1:4, w/w. The composition may further comprise sufficient material to provide a dose of from 0.01 to 250mg of a compound of formula (II) per kg of animal body weight per dose.

Compositions in accordance with the invention enable the stability of the avermectin/milbemycin compound to be improved, even in the presence of certain excipients, such as foodstuffs or food extracts, which could otherwise cause degradation of the avermectin/milbemycin.

In one aspect, the composition is formulated in such a way as to delay release of the active avermectin/milbemycin compound *in vivo.* Release of the active compound may be delayed by inclusion of a polymer, in particular a cellulose derivative such as ethyl cellulose or a synthetic polymer such as Eudragit, which is a mixed copolymer of methacrylic acid and methyl methacrylate and has a mean molecular weight of about 135,000. "Eudragit" is a Trade Mark of Röhm Pharma GmbH.

A further aspect of the invention provides a process for the preparation of a composition in accordance with the invention, which process comprises preparing a mixture of at least one avermectin or milbemycin, at least one cyclodextrin, and, optionally, a solvent or mixture of solvents, and then removing the solvent or mixture of solvents if present, for example by freeze-drying, spray drying, filtration and/or evaporation. The complex thus formed is then optionally admixed with one or more excipients as defined above and/or shaped to form a bolus, tablet or capsule.

Suitable solvent-free methods in accordance with the invention include grinding and kneading.

Suitable solvents include polar solvents such as methanol. The preparation of the mixture is suitably carried out for example at ambient temperature. Evaporation of solvent may be carried out under reduced pressure, for example at about 60°C.

The following Examples illustrate the invention.

### Example 1

### Methyl b-cyclodextrin/milbemycin complex

Methyl-b-cyclodextrin (randomly methylated) and milbemycin (the compound of Example 6 of EP-A-0 421 568, mixture of E- and Z-isomers) were weighed out in the ratio 4:1. Each was dissolved in 20 ml of methanol and the two solutions combined in a 100 ml round bottomed flask. The methanol was then removed under reduced pressure at 60°C under constant rotation on a Buchi Rotavapor. The material obtained was removed from the flask and transferred to a 100 ml beaker and left under vacuum for a further 30 mins at 60°C to remove any residual traces of methanol. The material was then ground in a pestle and mortar to obtain a fine powder.

Milbemycin complexed as described in Example 1 was compared with the uncomplexed milbemycin with respect to its rate of degradation. Shelf life predictions were obtained using the Arrhenius method. The results are reported below:

### Uncomplexed milbemycin

| **Time (weeks)** | **50°C** | **25°C** |
|---|---|---|
| 8.0 | 43.7% | |
| 12.0 | | 96.2% |

* Shelf life prediction: **30 weeks** (6-153) [>99.9% significance]

### Complex of Example 1

| **Time (weeks)** | **50°C** | **25°C** |
|---|---|---|
| 8.0 | 95.0% | |
| 12.0 | | 100.8% |

* Shelf life prediction: **not possible: no significant decomposition at 25°C.**

* Shelf life equals period required for 10% potency loss at 25°C.

### Example 2

### β-Cyclodextrin/milbemycin complex

7g of milbemycin was weighed and transferred to a ceramic mortar to which 63g of β-cyclodextrin was weighed and added. The two powders were then mixed with a spatula until reasonably homogeneous and then ground with a pestle for at least 45 minutes. The product obtained is a complex of milbemycin and β-cyclodextrin.

### Example 3

### β-Cyclodextrin/milbemycin complex

7g of milbemycin was weighed and transferred to a 1000 ml steel vessel to which 63g of β-cyclodextrin was weighed and added. To the vessel were added sufficient steel ball bearings to ensure efficient grinding. The vessel is then sealed and rotated at a constant velocity (*ca* 30 r.p.m.) for one hour. The vessel is then opened and the material removed. The product is a complex of milbemycin and β-cyclodextrin.

### Example 4

| **Palatable pet tablets** | |
|---|---|
| **Ingredient** | **% (**^{**w**}**/w)** |
| Pet Tab Granules | |
| Complex of Example 2 or 3 | 1.40 |
| Epsiprantel | 7.69 |
| Desiccated Liver | 20.78 |
| Fish Meal | 15.59 |
| Protex 111 | 0.52 |
| Starch 1500 | 5.20 |
| Lactose BP | 16.47 |
| Avicel PH102 | 15.59 |
| Texapon L100 | 0.26 |
| | (83.50) |

| **Compression Mix** | |
|---|---|
| Pet Tab Granules | 83.50 |
| Aerosil 200 | 0.50 |
| Avicel PH102 | 15.00 |
| Magnesium Stearate | 1.00 |
| | (100.00) |

### Pet Tablet Granules

1) Weigh out all materials
2) Dissolve Texapon in deionised water to give a solution of 2.5% w/v.
3) Pass all other materials through 2mm sieve screen into a planetary mixer bowl.
4) Mix materials in planetary mixer until homogeneous.
5) Granulate material with Texapon solution and further deionised water until granulate of correct consistency is obtained.
6) Dry granules at 60°C until moisture content is below 5% (Karl-Fischer).
7. Weigh granules and calculate the amount of extra granular materials needed.

### Compression Mix

1) Weigh out pet tab granules and all extra granular materials.
2) Pass all extra granular materials through 500µm sieve.
3) Mix extra granular materials and pet tab granules in a planetary mixer until homogeneous.
4) Proceed to compression.

### Example 5

| **Ingredient** | **% (**^{**w**}**/w)** |
|---|---|
| **Pet Tab Granules** | |
| Epsiprantel | 7.69 |
| Desiccated Liver | 20.78 |
| Fish Meal | 15.59 |
| Protex 111 | 0.52 |
| Starch 1500 | 5.20 |
| Lactose BP | 16.47 |
| Avicel PH102 | 15.59 |
| Texapon L100 | 0.26 |
| | (82.10) |

| **Compression Mix** | |
|---|---|
| Pet Tab Granules | 82.10 |
| Complex of Example 2 or 3 | 1.40 |
| Aerosil 200 | 0.50 |
| Avicel PH102 | 15.00 |
| Magnesium Stearate | 1.00 |
| | (100.00) |

Method: as for Example 4

## Claims

1. A pharmaceutical composition comprising at least one anthelmintically active compound which is an avermectin or milbemycin, in the form of a complex with at least one cyclodextrin.

2. A composition as claimed in Claim 1 in which the avermectin or milbemycin is milbemycin, ivermectin, doramectin, moxidectin, nemadectin, abamectin or a compound of the partial formula (I) 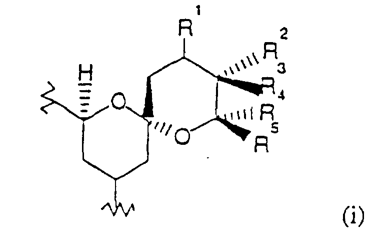 wherein R¹ is an optionally substituted amino or imino group, such as optionally O-substituted oxyimino,optionally N-substituted hydrazone or optionally N-substituted semicarbazone, and R² to R⁵ are the same or different and each is hydrogen or an organic radical.

3. A composition as claimed in claim 2 in which the compounds of formula (i) are compounds of formula (I) 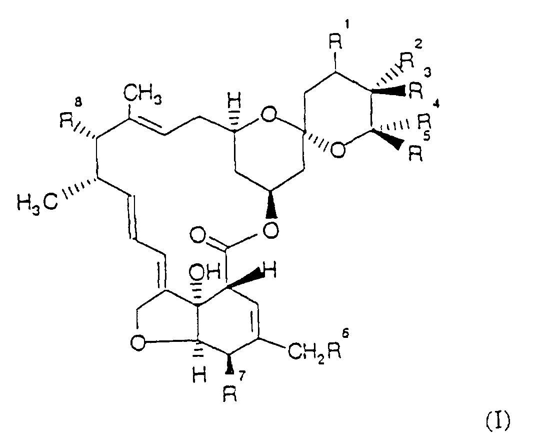 wherein R¹ to R⁵ are as defined in claim 2, R⁶ is hydrogen or optionally protected hydroxy; R⁷ is alkoxy, optionally protected hydroxy, oxo or optionally O-substituted oxyimino; and R⁸ is hydrogen, optionally protected hydroxy, or a group 4' -(a-L-oleandrosyl)-a-L-oleandrosyloxy or a-L-oleandrosyloxy wherein the terminal hydroxy group is optionally protected.

4. A composition as claimed in claim 3 wherein R¹ is O-substituted oxyimino, R² to R⁴ are hydrogen, R⁵ is an organic radical, R⁶ and R⁸ are hydrogen, and R⁷ is hydroxy.

5. A composition as claimed in claim 3 wherein the protecting group for hydroxy is t-butyldimethylsilyl or acyl (alkanoyloxy).

6. A composition as claimed in claim 2 or 3 wherein when any of R² to R⁵ is an organic radical, it is C₁-C₂₀ alkyl; C₂-C₂₀ alkenyl; C₂-C₂₀ alkynyl; aryl; heterocyclyl; C₃-C₂₀ mono-, bi- and tri-cycloalkyl; C₄-C₂₀ mono-, bi- and tri-cycloalkenyl; or aralkyl; any alkyl, alkenyl or alkynyl moiety being optionally substituted by one or more substituents selected from the group consisting of hydroxy, alkoxy, alkylthio, oxo, halogen, trifluoromethyl, and optionally substituted amino; and wherein (a) the term 'aryl' means phenyl and naphthyl optionally substituted with up to five groups selected from halogen, C₁-C₆ alkyl, aryl, C₁-C₆ alkoxy, halo substituted (C₁-C₆) alkyl, hydroxy, amino, nitro, carboxy, C₁-C₆ alkoxycarbonyl, C₁-C₆ alkoxycarbonyl-(C₁-C₆)-alkyl, C₁-C₆ alkylcarbonyloxy, and C₁-C₆ alkylcarbonyl; and (b) the term 'heterocyclyl' means saturated, unsaturated and aromatic single or fused rings comprising up to four hetero atoms in the ring selected from oxygen, nitrogen and sulphur and optionally substituted with up to three halogen, C₁-C₆ alkyl, C₁-C₆ alkoxy, halo-(C₁-C₆)-alkyl, hydroxy, amino, carboxy, C₁-C₆ alkoxycarbonyl, C₁-C₆ alkoxycarbonyl(C₁-C₆) alkyl, aryl or oxo groups.

7. A composition as claimed in claim 1, wherein the anthelmintically active compound is 5-oximino-22,23-dihydro-25-cyclohexylavermectin B1 monosaccharide

8. A composition as claimed in any one of the preceding claims wherein the cyclodextrin is a-, b- or g-cyclodextrin, or a derivative or mixture thereof.

9. A composition as claimed in claim 8 wherein the derivative is a methyl b-cyclodextrin, hydroxyethyl b-cyclodextrin, or hydroxypropyl b-cyclodextrin, or a b-cyclodextrin/epichlorohydrin copolymer.

10. A composition as claimed in claim 8 wherein the cyclodextrin beta-cyclodextrin or dimethyl beta-cyclodextrin.

11. A composition as claimed in any one of the preceding claims wherein the ratio of avermectin/milbemycin(s) : cyclodextrin(s) is from 1:1 to 1:10 w/w.

12. The use of a composition as claimed in any one of the previous claims for the manufacture of a medicament for the treatment of helminthiasis of the human or non-human animal body.

13. The use of an anthelmintically effective amount of a pharmaceutical composition as claimed in any one of Claims 1 to 11 for the preparation of a medicament for the treatment of helminthiasis, particularly nematode infestations in domestic animals.

14. An animal food containing a composition as claimed in any one of claims 1 to 11.

15. A composition as claimed in any one of claims 1 to 11 which additionally comprises at least one compound with activity against tapeworm.

16. A process for the preparation of a composition as claimed in claim 1, which process comprises preparing a mixture of at least one avermectin or milbemycin, at least one cyclodextrin, and, optionally, a solvent or mixture of solvents, and then removing the solvent or mixture of solvents, if present.

17. A process as claimed in claim 16, wherein the solvent or mixture of solvents is removed by freeze drying, spray-drying, filtration and/or evaporation.

18. A process according to claim 16 or 17 wherein a composition as claimed in any one of claims 2 to 11 is prepared.

## Patentansprüche

1. Pharmazeutische Zusammensetzung mit mindestens einer anthelmintisch aktiven Verbindung, die ein Avermectin oder Milbemycin ist in Form eines Komplexes mit mindestens einem Cyclodextrin.

2. Zusammensetzung nach Anspruch 1, bei der das Avermectin oder Milbemycin Milbemycin, Ivermectin, Doramectin, Moxidectin, Nemadectin, Abamectin oder eine Verbindung der Teilformel (i) 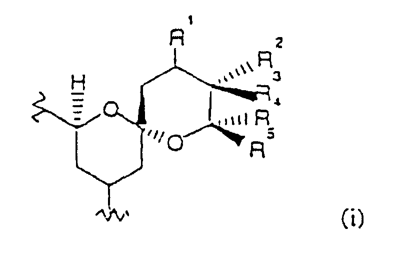 ist, worin R¹ eine gegebenenfalls substituierte Aminooder Iminogruppe ist, z.B. eine gegebenenfalls O-substituierte Oxyimino-, gegebenenfalls N-substituierte Hydrazon- oder gegebenenfalls N-substituierte Semicarbazongruppe, und R² bis R⁵ gleich oder verschieden sind und jeweils Wasserstoff oder ein organischer Rest sind.

3. Zusammensetzung nach Anspruch 2, worin die Verbindungen der Formel (i) Verbindungen der Formel (I) 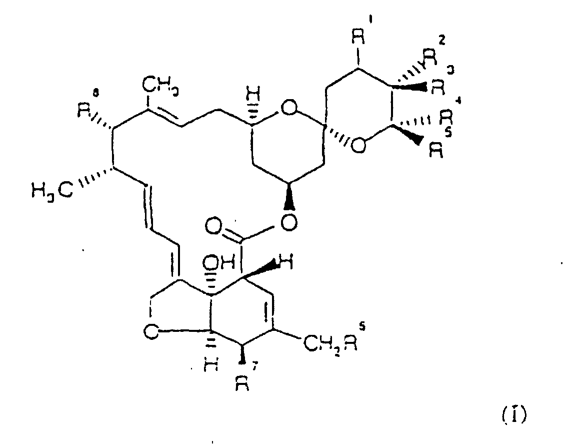 sind, worin R¹ bis R⁵ wie in Anspruch 2 definiert sind, R⁶ Wasserstoff oder ein gegebenenfalls geschützter Hydroxyrest ist; R⁷ ein Alkoxy-, gegebenenfalls geschützter Hydroxy-, Oxo- oder gegebenenfalls O-substituierter Oxyiminorest ist und R⁸ Wasserstoff, ein gegebenenfalls geschützter Hydroxyrest oder eine 4'-(a-L-Oleandrosyl)-a-L-oleandrosyloxy- oder a-L-Oleandrosyloxygruppe ist, beider die endständige Hydroxygruppe gegebenenfalls geschützt ist.

4. Zusammensetzung nach Anspruch 3, worin R¹ eine O-substituierte Oxyiminogruppe ist, R² bis R⁴ Wasserstoff sind, R⁵ ein organischer Rest ist, R⁶ und R⁸ Wasserstoff sind und R⁷ ein Hydroxyrest ist.

5. Zusammensetzung nach Anspruch 3, worin die Schutzgruppe für Hydroxy eine t-Butyldimethylsilyl- oder Acyl(alkanoyloxy)-Gruppe ist.

6. Zusammensetzung nach Anspruch 2 oder Anspruch 3, worin dann, wenn einer der Reste R² bis R⁵ ein organischer Rest ist, dieser ein C₁-C₂₀-Alkyl-, C₂-C₂₀-Alkenyl-, C₂-C₂₀-Alkinyl-, Aryl-, Heterocyclyl-, C₃-C₂₀-Mono-, Bi- und Tricycloalkyl-, C₄-C₂₀-Mono-, Bi- und Tricycloalkenyloder Aralkylrest ist, wobei der Alkyl-, Alkenyl- oder Alkinylanteil gegebenenfalls mit einem oder mehreren Substituenten substituiert sein kann, die ausgewählt sind aus der Gruppe bestehend aus Hydroxy-, Alkoxy-, Alkylthio-, Oxo-, Halogen-, Trifluormethyl- und gegebenenfalls substituierten Aminoresten und wobei (a) der Ausdruck "Arylrest" einen Phenyl- und Naphthylrest bedeutet, die gegebenenfalls mit bis zu 5 Gruppen substituiert sind ausgewählt aus Halogen-, C₁-C₆-Alkyl-, Aryl-, C₁-C₆-Alkoxy-, mit Halogen substituierten (C₁-C₆)-Alkyl-, Hydroxy-, Amino-, Nitro-, Carboxy-, C₁-C₆-Alkoxycarbonyl-, C₁-C₆-Alkoxycarbonyl-(C₁-C₆)-alkyl-, C₁-C₆-Alkylcarbonyloxy- und C₁-C₆-Alkylcarbonylresten und (b) der Ausdruck "Heterocyclylrest" gesättigte, ungesättigte oder aromatische einzelne oder kondensierte Ringe mit bis zu 4 Heteroatomen im Ring ausgewählt aus Sauerstoff, Stickstoff und Schwefel bedeutet, die gegebenenfalls mit bis zu 3 Halogen-, C₁-C₆-Alkyl-, C₁-C₆-Alkoxy-, Halogen-(C₁-C₆)-alkyl-, Hydroxy-, Amino-, Carboxy-, C₁-C₆-Alkoxycarbonyl-, C₁-C₆-Alkoxycarbonyl- (C₁-C₆) -alkyl-, Aryl- oder Oxogruppen substituiert sind.

7. Zusammensetzung nach Anspruch 1, worin die anthelmintisch aktive Verbindung 5-Oximino-22,23-dihydro-25-cyclohexylavermectin-B1-monosaccharid ist.

8. Zusammensetzung nach einem der vorhergehenden Ansprüche, worin das Cyclodextrin a-, b- oder g-Cyclodextrin oder ein Derivat oder eine Mischung davon ist.

9. Zusammensetzung nach Anspruch 8, wobei das Derivat ein Methyl-b-cyclodextrin, Hydroxyethyl-b-cyclodextrin oder Hydroxypropyl-b-cyclodextrin oder ein b-Cyclodextrin/Epichlorhydrin-Copolymer ist.

10. Zusammensetzung nach Anspruch 8, wobei das Cyclodextrin β-Cyclodextrin oder Dimethyl-β-cyclodextrin ist.

11. Zusammensetzung nach einem der vorhergehenden Ansprüche, wobei das Verhältnis von Avermectin/Milbemycin(en): Cyclodextrin(en) 1:1 bis 1:10 G/G ist.

12. Verwendung einer Zusammensetzung nach einem der vorhergehenden Ansprüche zur Herstellung eines Arzneimittels zur Behandlung von Helminthiasis des menschlichen oder nicht menschlichen tierischen Körpers.

13. Verwendung einer anthelmintisch wirksamen Menge einer pharmazeutischen Zusammensetzung nach einem der Ansprüche 1 bis 11 zur Herstellung eins Arzneimittels zur Behandlung von Helminthiasis, insbesondere von Nematodenbefall bei Haustieren.

14. Tierfutter enthaltend eine Zusammensetzung nach einem der Ansprüche 1 bis 11.

15. Zusammensetzung nach einem der Ansprüche 1 bis 11, die zusätzlich mindestens eine Verbindung mit einer Aktivität gegen den Bandwurm enthält.

16. Verfahren zur Herstellung einer Zusammensetzung nach Anspruch 1, wobei das Verfahren umfasst, dass eine Mischung aus mindestens einem Avermectin oder Milbemycin, mindestens einem Cyclodextrin und gegebenenfalls einem Lösungsmittel oder einer Mischung von Lösungsmitteln hergestellt wird und dann das Lösungsmittel oder die Mischung der Lösungsmittel, falls vorhanden, entfernt wird.

17. Verfahren nach Anspruch 16, wobei das Lösungsmittel oder die Mischung von Lösungsmitteln durch Gefriertrocknen, Sprühtrocknen, Filtration und/oder Verdampfen entfernt wird.

18. Verfahren nach Anspruch 16 oder Anspruch 17, wobei eine Zusammensetzung nach einem der Ansprüche 2 bis 11, hergestellt wird.

## Revendications

1. Composition pharmaceutique comprenant au moins un composé à activité anthelminthique qui consiste en une avermectine ou milbémycine, sous forme d'un complexe avec au moins une cyclodextrine.

2. Composition suivant la revendication 1, dans laquelle l'avermectine ou la milbémycine est la milbémycine, l'ivermectine, la doramectine, la moxidectine, la némadectine, l'abamectine ou un composé de formule partielle (i) 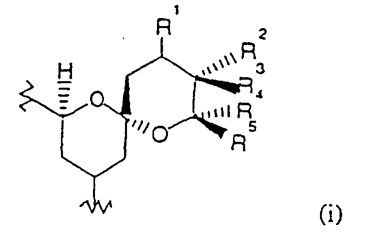 dans laquelle R¹ représente un groupe amino ou imino facultativement substitué, tel qu'un groupe oxyimino facultativement O-substitué, hydrazone facultativement N-substitué ou semicarbazone facultativement N-substitué, et R² à R⁵ sont identiques ou différents et chacun représente un atome d'hydrogène ou un radical organique.

3. Composition suivant la revendication 2, dans laquelle les composés de formule (i) sont des composés de formule (I) 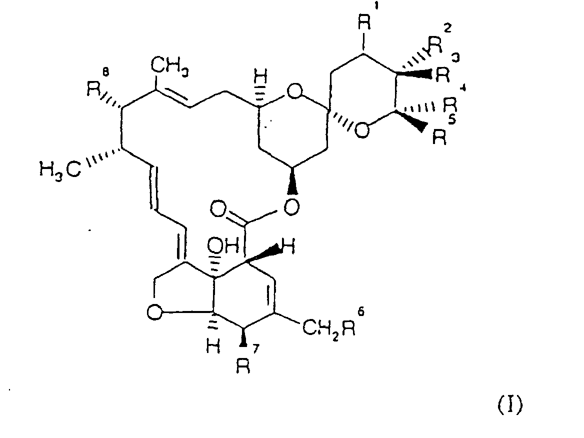 dans laquelle R¹ à R⁵ répondent aux définitions suivant la revendication 2, R⁶ représente un atome d'hydrogène ou un groupe hydroxy facultativement protégé ; R⁷ représente un groupe alkoxy, hydroxy facultativement protégé, oxo ou oxyimino facultativement O-substitué ; et R⁸ représente un atome d'hydrogène, un groupe hydroxy facultativement protégé, ou un groupe 4'-(α-L-oléandrosyl)-a-L-oléandrosyloxy ou α-L-oléandrosyloxy dans lequel le groupe hydroxy terminal est facultativement protégé.

4. Composition suivant la revendication 3, dans laquelle R¹ représente un groupe oxyimino O-substitué, R² à R⁴ représentent des atomes d'hydrogène, R⁵ représente un radical organique, R⁶ et R⁸ représentent des atomes d'hydrogène et R⁷ représente un groupe hydroxy.

5. Composition suivant la revendication 3, dans laquelle le groupe protecteur pour le groupe hydroxy est un groupe tertio-butyldiméthylsilyle ou acyl(alcanoyloxy).

6. Composition suivant la revendication 2 ou 3, dans laquelle, lorsque l'un quelconque de R² à R⁵ est un radical organique, il consiste en un radical alkyle en C₁ à C₂₀ ; alcényle en C₂ à C₂₀ ; alcynyle en C₂ à C₂₀ ; aryle ; hétérocycle ; mono-, bi- ou tri-cycloalkyle en C₃ à C₂₀ ; mono-, bi- ou tri-cycloalcényle en C₄ à C₂₀ ; ou aralkyle ; n'importe quel groupement alkyle, alcényle ou alcynyle étant facultativement substitué avec un ou plusieurs substituants choisis dans le groupe consistant en des substituants hydroxy, alkoxy, alkylthio, oxo, halogéno, trifluorométhyle et amino facultativement substitué ; et dans laquelle (a) le terme "aryle" désigne les groupes phényle et naphtyle facultativement substitués avec jusqu'à cinq groupes choisis entre des groupes halogéno, alkyle en C₁ à C₆, aryle, alkoxy en C₁ à C₆, alkyle en C₁ à C₆ à substituant halogéno, hydroxy, amino, nitro, carboxy, (alkoxy en C₁ à C₆)carbonyle, (alkoxy en C₁ à C₆) carbonyl-(alkyle en C₁ à C₆), (alkyle en C₁ à C₆) carbonyloxy et (alkyle en C₁ à C₆)carbonyle ; et (b) le terme "hétérocycle" désigne des noyaux uniques ou condensés saturés, insaturés et aromatiques comprenant jusqu'à 4 hétéroatomes dans le noyau choisi entre des atomes d'oxygène, d'azote et de soufre et facultativement substitués avec jusqu'à trois substituants halogéno, alkyle en C₁ à C₆, alkoxy en C₁ à C₆, halogénalkyle en C₁ à C₆, hydroxy, amino, carboxy, (alkoxy en C₁ à C₆) carbonyle, (alkoxy en C₁ à C₆)carbonyl(alkyle en C₁ à C₆), aryle ou oxo.

7. Composition suivant la revendication 1, dans laquelle le composé à activité anthelminthique est le monosaccharide de 5-oximino-22,23-dihydro-25-cyclohexyl-avermectine B1.

8. Composition suivant l'une quelconque des revendications précédentes, dans laquelle la cyclodextrine est une alpha-, bêta- ou gamma-cyclodextrine ou un de ses dérivés ou mélanges.

9. Composition suivant la revendication 8, dans laquelle le dérivé est une méthyl-bêta-cyclodextrine, hydroxyéthyl-bêta-cyclodextrine ou hydroxypropyl-bêta-cyclodextrine, ou un copolymère bêta-cyclodextrine/épi-chlorhydrine.

10. Composition suivant la revendication 8, dans laquelle la cyclodextrine consiste en bêta-cyclodextrine ou dimêthyl-bêta-cyclodextrine.

11. Composition suivant l'une quelconque des revendications précédentes, dans laquelle le rapport avermectine/milbémycine(s):cyclodextrine(s) est compris dans l'intervalle de 1:1 à 1:10 en poids/poids.

12. Utilisation d'une composition suivant l'une quelconque des revendications précédentes pour la production d'un médicament destiné au traitement de l'helminthiase de l'organisme de l'homme ou d'une espèce animale, autre que l'espèce humaine.

13. Utilisation d'une quantité à effet anthelminthique d'une composition pharmaceutique suivant l'une quelconque des revendications 1 à 11 pour la préparation d'un médicament destiné au traitement de l'helminthiase, en particulier d'infestations par des nématodes chez des animaux domestiques.

14. Aliment pour animaux, contenant une composition suivant l'une quelconque des revendications 1 à 11.

15. Composition suivant l'une quelconque des revendications 1 à 11, qui comprend en outre au moins un composé ayant une activité contre les cestodes.

16. Procédé pour la préparation d'une composition suivant la revendication 1, procédé qui comprend la préparation d'un mélange d'au moins une avermectine ou milbémycine, d'au moins une cyclodextrine et, facultativement, d'un solvant ou mélange de solvants, puis l'élimination du solvant ou mélange de solvants, s'il est présent.

17. Procédé suivant la revendication 16, dans lequel le solvant ou mélange de solvants est éliminé par lyophilisation, séchage par pulvérisation, filtration et/ou évaporation.

18. Procédé suivant la revendication 16 ou 17, dans lequel une composition suivant l'une quelconque des revendications 2 à 11 est préparée.
